# FASCICULE DE BREVET EUROPEEN

(11) **EP 0 724 841 B1**
(45) Date de publication et mention de la délivrance du brevet: **30.05.2001**
(21) Numéro de dépôt: 96400204.2
(22) Date de dépôt: 29.01.1996
(51) Int. Cl.: A23L 1/105, C12N 1/20

(54) **Composition nutritive résultant de la trempe du mais et son procédé d'obtention**
Maisquellwasser und Verfahren zu dessen Herstellung
Nutritional corn steep liquor and method for obtaining it

(30) Priorité: 31.01.1995 FR 9501110
(43) Date de publication de la demande: 07.08.1996
(73) Titulaire: ROQUETTE FRERES, 62136 Lestrem (FR)
(72) Inventeur: Saniez, Marie-Hélène, F-59350 Saint-Andre (FR); Gouy, Pierre-Antoine, F-59840 Perenchy (FR)
(74) Mandataire: Boulinguiez, Didier

(56) Documents cités:
- EP-A- 0 321 004
- EP-A- 0 379 232
- EP-A- 0 577 294
- CEREAL CHEMISTRY, vol. 68, no. 3, MINNEAPOLIS US, pages 319-320, XP002002667 S.R. ECKHOFF & C.C. TSO: "Starch recovery from steeped corn grits as affected by drying temperature and added commercial protease."
- CEREAL CHEMISTRY, vol. 68, no. 1, MINNEAPOLIS US, pages 7-12, XP000175097 J.D. STEINKE & L.A. JOHNSON: "Steeping maize in the presence of multiple enzymes. I. Static batchwise steeping."
- STARKE, vol. 26, no. 12, WEINHEIM DE, pages 433-436, XP002002669 H.M. EL-SAIED: "Influence of phytates on protease and dextrinogenic amylase formation."

## Description

La présente invention a pour objet une composition nutritive résultant de la trempe du maïs.

Elle vise également le procédé d'obtention d'une telle composition, ainsi que son application comme milieu de culture dans les industries de fermentation et comme aliment ou additif alimentaire dans des compositions destinées aux humains ou aux animaux.

La trempe du maïs constitue la première étape de l'extraction de l'amidon en amidonnerie humide. Elle consiste à maintenir le maïs placé dans des silos pendant un temps donné au sein d'une eau chaude contenant une faible quantité d'anhydride sulfureux, ceci afin de faciliter la séparation ultérieure protéines-cellulose-amidon, et d'empêcher par ailleurs la croissance de microorganismes indésirables.

Au cours de cette opération, deux phénomènes essentiels prennent place simultanément. D'une part, les matières solubles hautement fermentescibles contenues dans les grains de maïs sont transférées dans les eaux de trempe. D'autre part, les conditions de trempe (présence de sulfites, de sucres réducteurs, niveau de température) sont favorables au développement rapide de bactéries, lactiques principalement.

Le principal intérêt des eaux de trempe, appelées couramment ''corn-steep'' par l'homme du métier, tient à leur composition en composés essentiels qui sont issus du transfert de ces matières solubles. Ces composés constituent des facteurs favorables à la croissance des microorganismes comme à la production de métabolites secondaires, et font des eaux de trempe une source idéale de matières nutritives dans les industries de fermentation.

En effet, le corn-steep contient en tant que sources de carbone facilement assimilables : des sucres et des acides organiques, comme sources d'azote et de carbone : des amino-acides et des polypeptides et comme sources d'oligo-éléments nécessaires à la croissance des microorganismes : des agents "tampons" et des minéraux.

En outre, il constitue un substrat relativement peu coûteux, comparativement aux extraits de levures qui représentent la matière de référence dans ce domaine, et qui sont utilisés également en alimentation humaine et animale.

Par ailleurs, il est connu que l'utilisation du corn-steep en remplacement de sources d'azote complexes telles que les protéines de coton ou de soja, permet d'augmenter de manière substantielle les rendements de production d'antibiotiques.

Pour une utilisation en fermentation, le corn-steep doit être soumis au préalable à une stérilisation, dont les conditions de température et de pH ainsi que la durée sont choisies afin d'obtenir la destruction des microorganismes. Ainsi, la température est généralement comprise entre 105 et 130°C et le pH varie entre 3,0 et 8,0. Or, ces conditions de température et de pH provoquent la précipitation de certains constituants du corn-steep, ce qui entraîne de nombreux inconvénients.

En effet, l'utilisation d'un tel corn-steep non homogène pose un problème au niveau de sa préparation, et en particulier lors de sa concentration. En outre, sa mise en oeuvre entraîne la formation d'un dépôt important sur les parois des fermenteurs, dépôt qui risque de colmater les échangeurs de température. Enfin, la récupération des produits de fermentation peut être perturbée de manière importante suite au colmatage des membranes et des filtres.

Parmi les solutions proposées pour résoudre ce problème, la plus ancienne est celle qui consiste à extraire du corn-steep, par précipitation chimique, les substances responsables de la coagulation. Ainsi, l'addition au corn-steep d'agents alcalins (chaux, soude,...) ou de composés métalliques (sels d'aluminium notamment), afin de faire précipiter certaines protéines, les composés sulfite ou sulfate ou encore l'acide phytique, est connue. Cependant, outre le surcoût associé à ce traitement supplémentaire, l'inconvénient majeur en est l'élimination de substances nutritives du milieu. De plus, un tel traitement nécessite l'introduction dans le corn-steep de produits chimiques dans des quantités qui peuvent être importantes, ce qui modifie notablement sa composition et limite ainsi ses utilisations potentielles.

Il a également été fait appel à l'ultrafiltration pour séparer des eaux de trempe du maïs les espèces moléculaires, notamment protéines et peptides, coagulables à la chaleur. Cette technique a d'ailleurs fait l'objet d'un brevet français N° 2 140 672 délivré à la Société SCHOLTEN-HONIG RESEARCH.

Enfin, il a été proposé plus récemment de traiter le corn-steep par des enzymes.

Ainsi, le brevet américain N° 4 914 029 délivré à la Société DORR-OLIVER décrit un traitement par un mélange phytase-cellulase.

Cependant, si un tel traitement permet d'éviter la précipitation de l'acide phytique, il ne suffit pas à résoudre totalement le problème de la formation d'un précipité lors de la stérilisation du corn-steep.

Par ailleurs, un traitement par une protéase est décrit dans le brevet japonais N° 04-198 080 déposé par la Société MITSUI TOATSU CHEMICALS : l'action d'une telle enzyme sur un corn-steep contribue à l'amélioration de sa filtrabilité. Le corn-steep ainsi traité est destiné à la préparation d'un engrais liquide.

De même, M. ROUSHDI, Y. GHALI et A. HASSANEAN ont étudié l'action de deux enzymes protéolytiques (l'Alcalase et la Neutrase fabriquées par la Société NOVO) sur les eaux de trempe du maïs afin de réduire la durée de l'opération de trempe et d'obtenir un amidon ayant une teneur réduite en protéine.

Toutefois, il est apparu qu'un traitement du corn-steep avec une enzyme protéolytique n'était pas suffisant pour écarter de manière satisfaisante les problèmes évoqués précédemment.

Par conséquent, aucun des traitements décrits et mis en oeuvre jusqu'à présent n'a permis de résoudre de manière satisfaisante le problème posé par la formation d'un précipité lors de la stérilisation du corn-steep, c'est-à-dire sans entraîner la perte d'une fraction non négligeable des constituants du corn-steep.

Or, la demanderesse vient de mettre au point un corn-steep dont les qualités nutritionnelles sont intactes, voire améliorées, et qui résiste au traitement thermique nécessité par son utilisation comme milieu de fermentation, ou comme additif en alimentation humaine ou animale. En particulier, la formation d'un précipité lors de la stérilisation du corn-steep est considérablement réduite, à un niveau qu'aucune des solutions proposées jusqu'alors n'avait permis d'obtenir.

En outre, cette composition nutritive présente l'avantage de pouvoir être concentrée au delà de 60 % de matière sèche sans se heurter à un phénomène de prise en masse, comme dans le cas des corn-steep de l'art antérieur. Une telle concentration permet de conférer à la composition nutritive selon l'invention une très bonne stabilité et une viscosité parfaitement adaptée aux conditions industrielles de mise en oeuvre, telles que notamment le transfert par des pompes. Elle présente également un avantage économique, dû à une réduction des coûts de stockage et de transport, mais également des coûts engendrés par l'étape d'évaporation. En effet, le colmatage des évaporateurs de corn-steep au niveau des réchauffeurs représente un problème important, dont la seule solution apportée aujourd'hui consiste en un nettoyage des évaporateurs et des tuyauteries par mise en oeuvre de soude et d'acide nitrique.

Cet avantage économique est encore appréciable au niveau de l'étape de séchage d'aliments secs pour les animaux, obtenus à partir d'une composition selon l'invention, par exemple par incorporation de celle-ci sur des drèches de maïs sèches.

A l'avantage économique s'ajoute dans ce cas un intérêt écologique puisque les nuisances olfactives associées au séchage sont notablement réduites.

L'invention se rapporte donc en premier lieu à une composition nutritive caractérisée par le fait qu'elle présente un rapport concentration en phosphore inorganique sur concentration en phosphore total compris entre 35 et 95 %, et que le dosage des protéines qu'elle contient, réalisé selon un test C, donne une valeur inférieure ou égale à 5.

Les concentrations en phosphore inorganique et phosphore total sont mesurées selon des méthodes connues et telles que celles décrites ci-après.

En ce qui concerne le phosphore inorganique, la méthode de référence consiste à mesurer l'absorbance, à une longueur d'onde de 360 nm, d'un complexe obtenu par réaction entre le phosphore inorganique et le molybdate d'ammonium, absorbance directement proportionnelle à la quantité de phosphore inorganique présent dans l'échantillon. Pour la mise en oeuvre de cette méthode, on peut utiliser par exemple le kit de dosage vendu par la Société GILFORD DIAGNOSTICS sous la référence 722058.

En ce qui concerne le phosphore total, son dosage est réalisé selon la méthode norme ISO 3946, qui repose sur le même principe que celui appliqué à la détermination de la concentration en phosphore inorganique, décrit ci-dessus. Une étape préalable est toutefois mise en oeuvre, étape qui consiste à détruire les matières organiques des produits à doser, par minéralisation à l'aide d'un mélange sulfonitrique et transformation des phosphates en orthophosphates. Les étapes suivantes consistent à former le complexe molybdique, puis à mesurer l'absorbance à une longueur d'onde de 825 nm.

De préférence, le rapport concentration phosphore inorganique sur concentration en phosphore total (Pi/Pt) est compris entre 60 et 95 %, et de manière encore plus préférentielle, entre 75 et 85 %.

En ce qui concerne le test C appliqué aux compositions selon l'invention, celui-ci a pour but de mesurer la concentration en protéine pour 100 grammes de matière sèche du surnageant de ces compositions.

Pour ce faire, on réalise une mesure spectrophotométrique de ces surnageants mis en présence d'un réactif coloré selon la méthode Bradford, méthode connue pour le dosage des protéines et décrite notamment dans Analysis Biochemistry (1976) 72, 248.

Dans le cas présent, le réactif mis en oeuvre est le Coomassie Brilliant Blue G 250, fabriqué par la Société PIERCE sous la référence 23200, et qui a la particularité de se lier aux protéines et aux peptides de plus de 20 amino-acides en solution acide, cette liaison s'accompagnant d'un changement de couleur du brun rouge au bleu.

Par mesure de l'absorbance à une longueur d'onde de 595 nm, absorbance correspondant au taux de réactif lié aux protéines présentes et par conséquent, proportionnelle à la quantité de protéines, puis comparaison avec une courbe étalon obtenue pour différentes concentrations d'une protéine standard (Bovine Serum Albumine, Réf. 23209 chez PIERCE), on détermine la concentration en protéine des surnageants des compositions.

Pour réaliser cette mesure, on amène au préalable la composition nutritive à tester à une matière sèche de 30 % en poids. Celle-ci est ensuite centrifugée à 5000 g durant 15 mn et le surnageant est récupéré, et amené à une matière sèche de 25 % en poids. On applique alors la méthode de Bradford à ce surnageant par introduction du réactif coloré, mélange, puis lecture de l'absorbance.

La valeur obtenue, correspondant à la concentration en protéine en grammes par litre de surnageant, est alors convertie en une concentration en gramme pour 100 g de matière sèche de surnageant.

Les compositions nutritives objet de la présente invention sont telles que la concentration en protéine en grammes pour 100 g de matière sèche de surnageant est inférieure ou égale à 5.

L'invention se rapporte en second lieu à un procédé d'obtention d'une composition nutritive résultant de la trempe de maïs qui possède les caractéristiques mentionnées ci-dessus.

Ce procédé consiste à traiter les eaux de trempe du maïs avec au moins une protéase et au moins une phytase.

De manière surprenante et inattendue, la Société Demanderesse a mis en évidence qu'un traitement combiné avec au moins une protéase et au moins une phytase donnait des résultats particulièrement intéressants auxquels, en toute logique, l'homme du métier ne pouvait s'attendre. En effet, les résultats d'un tel traitement ne correspondent pas à la simple addition des effets obtenus à la suite du traitement avec une protéase et de ceux obtenus après traitement avec une phytase.

La Société Demanderesse a ainsi pu démontrer qu'une réelle synergie résultait du traitement combiné des eaux de trempage du maïs avec de telles enzymes.

Le procédé selon l'invention consiste à introduire au moins une protéase et au moins une phytase, simultanément ou successivement, dans l'eau de trempe du maïs, à les laisser agir sous agitation pendant une durée qui dépend du type d'enzymes et des quantités mises en oeuvre, à suivre par prélèvements l'évolution au cours du temps du rapport Pi/Pt et la concentration en protéines, à inactiver ensuite ces enzymes, puis à concentrer la composition résultante par évaporation.

La première étape du procédé selon l'invention consiste donc en un traitement enzymatique.

Ce traitement s'effectue à la suite de l'étape de la trempe du maïs, sur une eau de trempe présentant une matière sèche comprise entre 5 et 50 %, et de préférence entre 10 et 20 %, un pH se situant entre 3,0 et 5,0, et une température variant entre 40 et 60°C.

Selon un mode préférentiel de réalisation de l'invention, le traitement enzymatique s'appliquera à des eaux de trempe obtenues dans les conditions décrites dans le brevet français n° 79 22106, qui présentent déjà une composition favorable à une utilisation ultérieure dans les industries de fermentation.

L'ordre selon lequel les enzymes sont introduites importe peu : ainsi, le traitement à la protéase peut précéder ou faire suite au traitement à la phytase. De même les deux types d'enzymes peuvent être mis en oeuvre simultanément.

Les quantités d'enzymes utilisées dépendent de l'activité propre à l'enzyme choisie et des conditions de sa mise en oeuvre (type de substrat, concentration du substrat, pH, température, durée du traitement).

Ces quantités sont comprises entre 0,1 et 2,0 % par rapport à la matière sèche du milieu en ce qui concerne la protéase et entre 0,01 % et 0,1% en ce qui concerne la phytase, ce qui correspond à une plage de 0,06 UA à 1,2 UA pour 100 g de matière sèche du milieu en ce qui concerne la protéase, et de 50 à 500 UP pour 100 g de matière sèche du milieu en ce qui concerne la phytase.

L'Unité Anson (UA) est l'unité protéasique, définie comme étant la quantité d'enzyme qui, à la température et au pH optimum de celle-ci, hydrolyse l'hémoglobine pour produire 1 micromole de Tyrosine par minute selon la méthode colorimétrique mettant en oeuvre le réactif de Folin Clocalteu. (Pour une description détaillée de cette méthode, on peut se référer à l'article de M.L. ANSON publié en 1939 dans J. Gen. Physiol 22, 79-89).

L'Unité Phytasique (UP) est définie comme correspondant à la quantité d'enzyme qui, à un pH de 5,5 et une température de 37°C, libère une micromole de phosphore inorganique par minute, à partir d'une solution de phytate de sodium à 0,0015 mole/litre. (Pour une description détaillée de cette méthode, on peut se référer à la demande internationale de brevet n° 93/16175).

La durée du traitement enzymatique varie entre 4 et 16 heures.

Le traitement est de préférence effectué sous agitation constante.

Les protéases utilisables dans le procédé selon l'invention sont: choisies en particulier parmi les protéases acides, telles que celles fabriquées par la Société BIOCON (ACID PROTEASE L B 59), par la Société GIST BROCADES (PROTEASE A), par la Société RÖHM (COROLASE PS), par la Société GENENCOR (PROTEASE B99), ou encore par la Société NOVO (FLAVOURZYME).

Le traitement protéase peut également résulter de la présence-même, au sein de l'eau de trempe, de protéases endogènes, c'est-à-dire générées par les bactéries qui se sont développées au cours de l'opération de trempe.

En ce qui concerne les phytases, on peut citer à titre d'exemple FINASE fabriquée par la Société ALKO, NATUPHOS 5000 commercialisée par la Société BASF, ou NOVO PHYTASE commercialisée par la Société NOVO.

Les phytases et protéases commerciales se présentent sous forme de préparations enzymatiques d'origine fongique qui possèdent de ce fait de activités enzymatiques secondaires telles que phosphatase, cellulase et lipase.

Ces enzymes "parasites", et en particulier les phosphatases, participent avantageusement au traitement de l'eau de trempe du maïs.

Des prélèvements du milieu réactionnel sont réalisés à intervalles réguliers afin de déterminer les concentrations en phosphore inorganique et phosphore total, ainsi que la concentration en protéine par mise en oeuvre du test C.

Lorsque le rapport Pi/Pt atteint la valeur minimale de 35 % et que la concentration en protéine déterminée selon le test C devient inférieure ou égale à 5, les réactions enzymatiques peuvent être stoppées par inactivation des enzymes.

Pour cela, on a recours à des moyens physiques (température) et/ou chimiques (pH). De préférence, on soumet le milieu réactionnel à un chauffage à 80-100°C pendant un temps compris entre 10 et 30 minutes.

Afin d'éliminer les bactéries et afin d'obtenir ainsi un produit limpide, la composition peut être centrifugée ou filtrée sur terre ou sur membrane de micro-filtration ou d'ultrafiltration.

Le procédé objet de la présente invention permet ainsi l'obtention d'un produit dont la matière sèche peut atteindre 65 %, ce qui présente nombre d'avantages comme cela a été décrit précédemment.

Les compositions selon l'invention, grâce à leurs caractéristiques nutritionnelles, présentent un intérêt particulier lorsqu'elles sont utilisées comme substrat de fermentation. Elles constituent en effet un substrat satisfaisant à la production dans de bonnes conditions de levures, de bactéries lactiques, ou d'autres microorganismes, mais également d'enzymes, d'antibiotiques, d'amino-acides, d'acides organiques, de vitamines, ou de biopesticides.

Elles sont également particulièrement adaptées à la production de métabolites obtenus par des microorganismes génétiquement modifiés.

En outre, elles intéressent l'industrie alimentaire, pour leurs propriétés nutritionnelles et pour leurs propriétés aromatiques, et sont ainsi utilisables en tant qu'aliment ou en tant qu'exhausteurs de goût dans des compositions destinées à l'alimentation humaine ou animale.

Les exemples donnés ci-après viennent illustrer l'invention sans toutefois la limiter.

### EXEMPLE 1 :

### Fabrication d'une composition nutritive selon l'invention

Dans un réacteur de 2 litres, équipé de moyens d'agitation et de régulation de la température, 1,5 litre de corn-steep à 200 g/l de matière sèche sont introduits. La température du substrat est ajustée à 50°C et l'agitation à 350 tr/mn. Le pH du produit n'est pas ajusté et reste donc le pH naturel du corn-steep, soit environ 4,5.

A ce substrat sont ajoutés :
- la phytase NATUPHOS 5000 (BASF), à un taux de 0,025 % par rapport à la matière sèche du substrat,
- la protéase ACID PROTEASE L B 59 fabriquée par la Société BIOCON, à la dose de 2% par rapport à la matière sèche du substrat.

La durée de réaction est de 16 heures, au cours de laquelle on suit l'évolution du rapport Pi/Pt et la concentration des protéines par prélèvement d'échantillons du milieu réactionnel toutes les 4 heures.

Les réactions enzymatiques sont alors inactivées par un chauffage à 90°C pendant 20 minutes puis refroidissement à température ambiante. Le produit résultant est alors centrifugé à 20°C à une vitesse de 4500 g pendant 15 minutes. Le culot, essentiellement constitué de bactéries lactiques, est éliminé et le surnageant est concentré sur un évaporateur de laboratoire jusqu'à une matière sèche de 60 %. Le produit obtenu est ensuite refroidi jusqu'à la température ambiante sous agitation.

La composition obtenue selon le procédé décrit ci-dessus présente un rapport Pi/Pt de 80 %.

Le dosage des protéines réalisé selon le test C donne une valeur de 2,2.

### EXEMPLE 2 :

### Fabrication de trois compositions A, B et C selon l'art antérieur

- La composition A correspond à un corn-steep témoin à 200 g/l de matière sèche qui n'a subi aucun traitement enzymatique.
- La composition B est obtenue selon le procédé décrit ci-dessus, à ceci près que seule la phytase NATUPHOS 5000 (BASF) a été ajoutée au substrat, à un taux de 0,025 % par rapport à la matière sèche.
- La composition C est obtenue selon le procédé décrit ci-dessus, à ceci près que seule la protéase ACID PROTEASE L B 59, fabriquée par BIOCON, a été ajoutée au substrat à un taux de 2 % par rapport à la matière sèche du substrat.

Les rapports Pi/Pt et les concentrations en protéine mesurées selon le test C de chacune des compositions A, B et C sont donnés ci-après.

| | A | B | C |
|---|---|---|---|
| Pi/Pt | 20 | 80 | 20 |
| Test C | 8,57 | 9,6 | 2,2 |

Ces trois compositions sont ensuite concentrées jusqu'à une matière sèche de 50 %, ce qui correspond à la valeur maximum que l'on puisse atteindre avec ces compositions.

### EXEMPLE 3 :

### A- Mise en évidence de la stabilité physique des compositions nutritives selon l'invention

La composition nutritive selon l'invention telle qu'obtenue à l'Exemple 1 est soumise à un test de décantation, comparativement aux trois compositions A, B et C décrites dans l'Exemple 2.

Ce test consiste à mesurer la hauteur du culot après décantation d'une composition préalablement stérilisée.

La composition selon l'Exemple 1 ainsi que les compositions A, B et C sont d'abord diluées à 25 g/litre, puis leur pH est ajusté à une valeur de 7 par addition d'une solution de soude 1N. Cette valeur de pH correspond à un niveau maximum de coagulation des protéines.

Ces compositions sont alors soumises à une stérilisation par chauffage à une température de 120°C durant 20 minutes. Elles sont ensuite refroidies, homogénéisées puis introduites dans des éprouvettes graduées dans lesquelles on-les laisse décanter.

Pour chacune des 4 compositions, la hauteur du culot est mesurée après 30 minutes et après 16 heures.

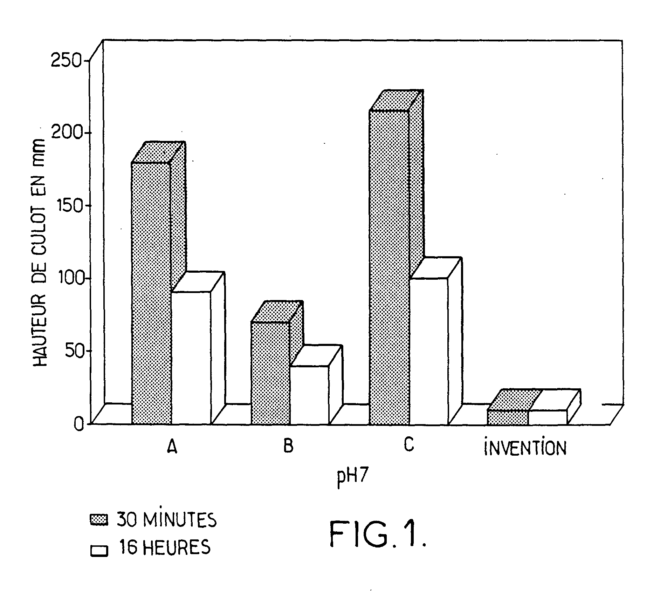

Les résultats obtenus, reportés sur la figure 1, sont les suivants :

| Composition | 30 mn | 16 heures |
|---|---|---|
| Invention | 10 | 10 |
| A | 180 | 90 |
| B | 70 | 40 |
| C | 215 | 100 |

A la lecture de ces résultats, la supériorité de la composition selon l'invention apparaît nettement. En effet, le culot est considérablement réduit par rapport à celui observé après stérilisation des compositions selon l'art antérieur, qui n'ont subi aucun traitement enzymatique, ou les traitements appliqués jusqu'alors, c'est-à-dire avec une phytase non associée à une protéase, ou avec une protéase seule.

L'intérêt des compositions selon l'invention au niveau de leur stabilité vis à vis d'un traitement de stérilisation est ici clairement démontré.

### B- Mesures comparatives des viscosités d'une composition selon l'invention et de celles des trois compositions selon l'art antérieur.

La composition nutritive selon l'invention, telle qu'obtenue à l'Exemple 1, ainsi que les trois compositions A, B et C décrites dans l'Exemple 2, ont fait l'objet d'une mesure d'une viscosité à l'aide du viscosimètre BROOKFIELD et selon le protocole d'utilisation de cet appareil.

La viscosité a été mesurée à une matière sèche de 50 %, donc après dilution préalable de la composition selon l'invention.

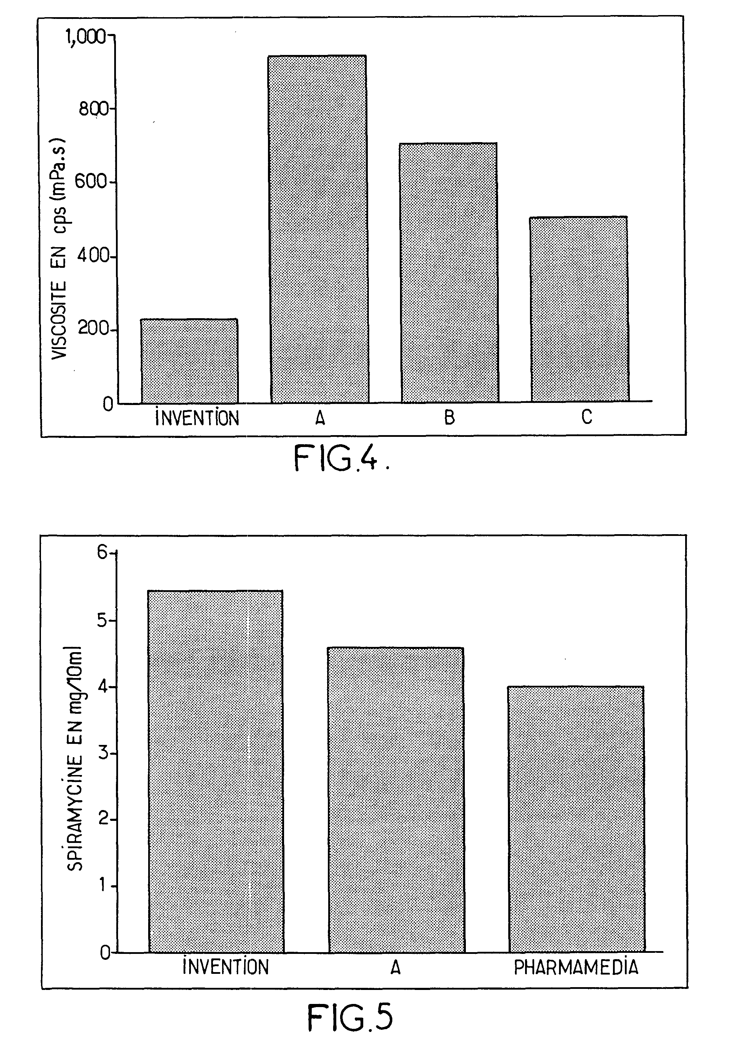

Les résultats, reportés sur la figure 4, sont donnés ci-après.

| Composition | Viscosité en milliPascal.sec |
|---|---|
| Invention | 230 |
| A | 940 |
| B | 700 |
| C | 500 |

En outre, des mesures comparatives de viscosité ont été réalisées à 55 % et 60 % de matière sèche entre la composition selon l'invention décrite dans l'Exemple 1 et la composition A décrite dans l'Exemple 2.

Les résultats sont les suivants :

| Composition | Viscosité en milliPascal.sec | |
|---|---|---|
| | à 55 % MS | à 60 % MS |
| Invention | 600 | 3.400 |
| A | 13.550 | aspect solide |

Ces résultats démontrent clairement la supériorité des compositions selon l'invention dans leur aptitude à être concentrées tout en restant adaptées à une mise en oeuvre industrielle.

### EXEMPLE 4 :

### Mise en évidence de la bonne filtrabilité des compositions nutritives selon l'invention

La composition nutritive selon l'invention, telle qu'obtenue, à l'Exemple 1 est soumise à un test de filtration, comparativement aux compositions A, B, C décrites dans l'Exemple 2.

Ce test consiste en une mesure du temps nécessaire pour que 10 ml de la solution à tester aient traversé un filtre constitué d'un disque en papier sans cendre, de 150 mm de diamètre (PROLABO - Référence 08.313.766).

La matière sèche des solutions à tester est ajustée à 25 g/l.

Ces solutions subissent, après dilution, une stérilisation par traitement thermique à 120°C pendant 20 minutes.

Leur pH correspond au pH naturel, soit 4,5.

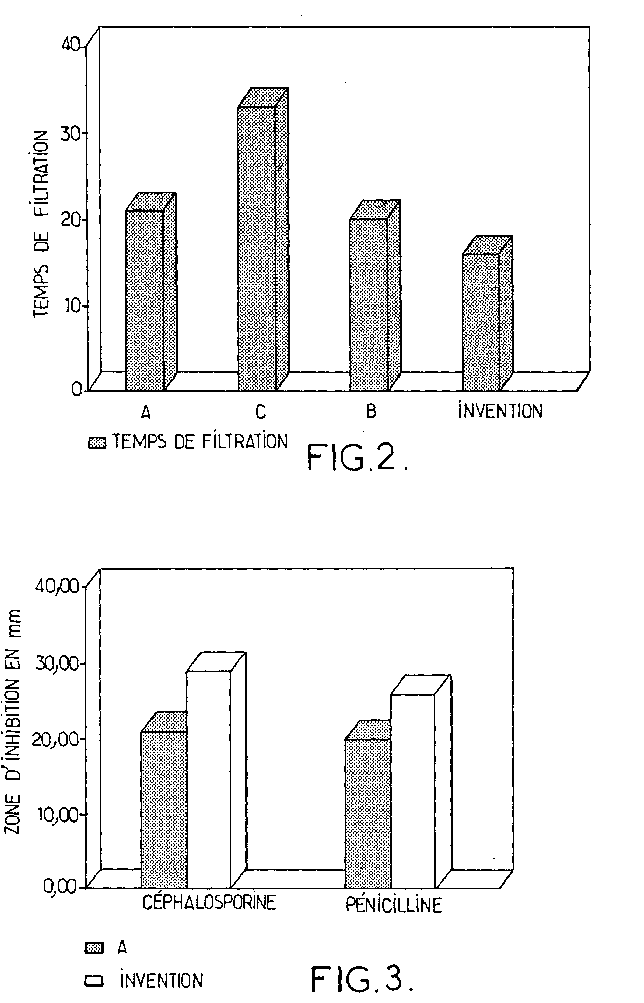

Les valeurs obtenues, reportées sur la figure 2, sont les suivantes :

| Composition | Temps de filtration |
|---|---|
| Invention | 16 |
| A | 21 |
| B | 33 |
| C | 2 |

Le temps de filtration de la composition selon l'invention est nettement inférieur à celui obtenu avec chacune des 3 autres compositions, qu'il s'agisse de la composition témoin ou de celle n'ayant subi un traitement enzymatique qu'avec la seule phytase ou la seule protéase.

Ceci démontre bien l'avantage des compositions selon l'invention et l'efficacité du traitement enzymatique phytase + protéase même après le traitement thermique nécessaire à la stérilisation des compositions.

### EXEMPLE 5 :

### Application des compositions selon l'invention à la croissance de microorganismes.

Les trois études décrites ci-après consistent à suivre l'augmentation du nombre de cellules en fonction du temps dans des milieux de culture contenant des concentrations différentes d'une composition nutritive selon l'invention comparativement à un milieu de culture contenant les mêmes concentrations de la composition A, décrite dans l'Exemple 2, qui correspond à un corn-steep témoin n'ayant subi aucun traitement enzymatique.

### . La première étude concerne la levure Saccharomyces cerevisiae.

On prépare des milieux de culture par addition dans de l'eau déminéralisée de glucose à raison de 10 g/l et de la composition nutritive telle que décrite dans l'exemple 1 et dont la matière sèche a été ramenée à 50 %, à des concentrations respectives de 3 g/l et 10 g/l (ces concentrations correspondent à des équivalents azote respectifs de 0,1 g/l et 0,33 g/l). Simultanément et de la même façon, on prépare un milieu de culture contenant respectivement 3 et 10 g/l de la composition A, décrite dans l'Exemple 2.

On ensemence alors 100 ml de chacun de ces milieux avec 0,1 % en volume d'une préculture de la souche.

L'incubation est réalisée à 30°C sous une agitation de 280 tours par minute pendant 24 heures.

Les numérations sont effectuées aux temps 0, 8 heures et 24 heures sur milieu OGA (oxytétracycline - glucose - agar) commercialisé par BIOKAR DIAGNOSTICS.

Les résultats obtenus sont rassemblés dans les tableaux ci-après.

Milieu à 3 g/l de composition

| C.F.U./ml (colonies formant unité) | 0 h | 8 h | 24 h |
|---|---|---|---|
| A | 5,1.10² | 3,5.10⁶ | 2,8.10⁸ |
| Invention | 3,6.10² | 9,1.10⁶ | 2,1.10⁹ |

Milieu à 10 g/l de composition

| C.F.U./ml | 0 h | 8 h | 24 h |
|---|---|---|---|
| A | 2,8.10² | 5,8.10⁶ | 5,2.10⁸ |
| Invention | 3,5.10² | 1,4.10⁷ | 1,9.10¹⁰ |

Les résultats obtenus démontrent les qualités nutritives particulièrement intéressantes des compositions selon l'invention par rapport à un milieu à base de corn-steep ne présentant pas les caractéristiques revendiquées.

En effet, à une concentration de 3 g/l, le milieu contenant la composition nutritive revendiquée permet une multiplication des cellules 10 fois supérieure, après 24 heures d'incubation, à celui contenant une composition nutritive selon l'art antérieur.

A une concentration de 10 g/l, le facteur multiplicatif est d'environ 2 après 8 heures, et d'environ 50 après 24 heures d'incubation.

### . La seconde étude a été réalisée avec la souche Bacillus Subtilis dans des conditions identiques à celles qui viennent d'être décrites.

Les milieux de culture sont préparés par addition de glucose (10 g/l), de sels (Mg SO4 et KH2 PO4, tous deux à 0,05 g/l) et des compositions nutritives (composition selon l'invention décrite dans l'exemple 1 et dont la matière sèche a été ramenée à 50 %, et composition comparative A décrite dans l'exemple 2), à 2,6 g/l, ce qui correspond à un équivalent azote de 0,086 g/l.

Les conditions d'ensemencement et d'incubation sont identiques à celles décrites précédemment.

Les numérations sont effectuées aux temps 0, 8 et 24 heures sur milieu gélose trypticase soja (DIFCO).

Les résultats donnés ci-après, confirment les conclusions précédentes, à savoir le grand intérêt des compositions nutritives selon l'invention comme substrat de fermentation.

| C.F.U./ml | 0 h | 8 h | 24 h |
|---|---|---|---|
| A | 1,4.10² | 1,9.10⁶ | 1,7.10⁸ |
| Invention | 2,1.10² | 8,8.10⁶ | 1,8.10⁹ |

### . La troisième étude a été réalisée avec la souche Lactobacillus plantarum sur trois milieux de culture différents : le premier contenant une composition nutritive selon l'invention, le second contenant la composition A décrite dans l'exemple 2 et le troisième à base d'extrait de levure, qui constitue le milieu de référence pour la croissance des microorganismes.

Les deux premiers milieux de culture sont préparés dans des conditions identiques à celles décrites précédemment, par addition de glucose (10 g/l) et d'une composition nutritive (composition selon l'invention décrite dans l'Exemple 1 et dont la matière sèche a été ramenée à 50 %, et composition A décrite dans l'exemple 2), à 3 g/l, ce qui correspond à un équivalent azote de 0,1 g/l.

Le milieu à base d'extrait de levure est obtenu par addition dans de l'eau déminéralisée de l'extrait de levure BACTO YEAST EXTRACT commercialisé par DIFCO, dans des quantités correspondant à un équivalent azote de 0,1 g/l.

On ensemence alors 200 ml de chacun des trois milieux avec 0,1 % en volume d'une préculture de la souche.

L'incubation est réalisée à 45°C sous légère agitation (20 tours par minute) pendant 24 heures. Les numérations sont effectuées aux temps 0, 8 et 24 heures sur milieu MRS (MAN - ROGOSA - SHARP) commercialisé par BIOKAR DIAGNOSTICS.

Les résultats obtenus sont rassemblés dans le tableau ci-après.

| C.F.U./ml | 0h | 8h | 24h |
|---|---|---|---|
| Invention | 3,2.10² | 3,1.10⁶ | 5,9.10⁸ |
| Composition A | 3,7.10² | 8,3.10⁵ | 8,1.10⁷ |
| Extrait de levure | 3,0.10² | 3,1.10⁵ | 3,2.10⁷ |

Ces résultats démontrent une fois de plus les remarquables qualités nutritives des compositions selon l'invention par rapport à celles de l'art antérieur.

### EXEMPLE 6 :

### Application des compositions nutritives selon l'invention à la production d'antibiotiques

Les trois études réalisées ont consisté à évaluer la production de trois antibiotiques, la céphalosporine, la pénicilline et la spiramycine, en utilisant comme substrats une composition nutritive selon l'invention d'une part, et une composition nutritive selon l'art antérieur d'autre part.

Ces productions sont évaluées par la méthode de dosage par diffusion (antibiogramme) qui consiste à comparer le diamètre d'inhibition de croissance d'une souche sensible à cet antibiotique, obtenu avec l'échantillon dont on souhaite connaître la concentration en antibiotique, au diamètre obtenu avec une gamme de concentrations connues du même antibiotique.

### - La première étude, relative à la production de céphalosporine se décompose de la manière suivante :

La souche productrice : *Cephalosporium acremonium,* après une étape de préculture, est ensemencée à un taux de 2,6 % (volume / volume) dans un milieu de production constitué de 50 g/l de glucose, 100 g/l de la composition nutritive selon l'invention telle que décrite dans l'exemple 1 et dont la matière sèche a été ramenée à 50%, ainsi que de sels (KH₂PO₄ à 2,5 g/l et CaCO₃ à 5 g/l).

Simultanément, on procède de la même façon avec un milieu constitué de 50 g/l glucose, 100 g/l d'une composition nutritive de l'art antérieur telle que la composition A décrite dans l'exemple 2 et des mêmes sels aux mêmes concentrations.

Après incubation à 30°C pendant 120 heures et sous une agitation de 120 tours par minute, on centrifuge le temps nécessaire pour l'obtention d'un surnageant limpide, qui contient la céphalosporine produite.

Des disques buvard sont alors imbibés avec 65 µl de ces surnageants, et déposés dans une boîte de Pétri sur un milieu gélosé trypticase soja et ensemencé avec la souche *Staplylococcus aureus* ATCC 6538, sensible à la céphalosporine.

On mesure alors le diamètre des zones d'inhibition de croissance obtenues dans les 2 cas après incubation 24 heures à 30°C.

### . La seconde étude, relative à la production de pénicilline, fait appel en tant que souche productrice à Pénicillium chrysogenum.

Cette souche, après une étape de préculture, est ensemencée à un taux de 4 % (volume / volume) dans un milieu de production constitué de 120 g/l de lactose , 76 g/l de la composition nutritive selon l'invention telle que décrite dans l'exemple 1 et dont la matière sèche a été ramenée à 50 %, ainsi que de sels ((NH4)₂SO₄ à 10 g/l, CaC0₃ à 10 g/l, KH₂PO₄ à 0,5 g/l et K₂SO₄ à 5 g/l)).

Simultanément, on procède de la même façon avec un milieu de production constitué de 120 g/l de lactose, 76 g/l d'une composition nutritive de l'art antérieur telle que la composition A décrite dans l'exemple 2, ainsi que des mêmes sels aux mêmes concentrations.

Après incubation à 25°C pendant 6 jours et sous une agitation de 220 tours par minute, on centrifuge pendant le temps nécessaire à l'obtention d'un surnageant limpide, qui contient la pénicilline produite.

Comme précédemment, des disques buvard sont imbibés avec 100 µl de ces surnageants et déposés dans des boites de Pétri contenant un milieu gélose trypticase soja préalablement ensemencé avec la souche *Sarcina lutea* ATCC 9341 sensible à la pénicilline.

On mesure alors le diamètre des zones d'inhibition de croissance obtenues dans les 2 cas après incubation pendant 16 heures à 30°C.

Les résultats obtenus (exprimés en millimètres) pour chacune de ces 2 études sont les suivants :

| | Céphalosporine | Pénicilline |
|---|---|---|
| Composition A | 21,00 | 20,00 |
| Invention | 29,00 | 26,00 |

Ces résultats sont représentés sur la figure 3.

### . La troisième étude, relative à la production de spiramycine, fait appel en tant que souche productrice à Streptomyces ambofaciens (ATCC 15154).

Cette souche, après une étape de préculture, est ensemencée à un taux de 5 % (volume/volume) dans un milieu de production contenant 5 g/l d'extrait de levure, 20 g/l d'amidon soluble, 6 g/l d'huile de soja ainsi que des sels (2,5 g/l de KH₂ PO₄ et 5 g/l de CaCO₃).

On ajoute à ce milieu 80 g/l de la composition nutritive selon l'invention ou 80 g/l de la composition nutritive de l'art antérieur telle que la composition A décrite dans l'Exemple 2, ou encore une solution de protéine de coton telle que celle commercialisée par TRADERS PROTEIN sous l'appellation PHARMAMEDIA, correspondant à un équivalent azote de 2,6 g/l.

Après incubation pendant 120 heures à 30°C et sous une agitation de 120 tours par minute, on centrifuge pendant le temps nécessaire à l'obtention d'un surnageant limpide, qui contient la spiramycine produite.

Comme précédemment, des disques buvard sont imbibés avec 65µl de ces surnageants et déposés dans des boîtes de Pétri contenant un milieu gélose-caséine-soja préalablement ensemencé avec la souche *Bacillus subtilis* DSM 347 sensible à la spiramycine.

On mesure alors le diamètre des zones d'inhibition de croissance obtenues dans les deux cas après incubation pendant 24 heures à 30°C.

Les résultats obtenus, exprimés en mg/10 ml par rapport à une gamme étalon en spiramycine établissant la correspondance entre le diamètre de la zone d'inhibition et la concentration en antibiotique produit, sont les suivants :

| | |
|---|---|
| Composition A | 4,60 |
| Invention | 5,45 |
| PHARMAMEDIA | 4,00 |

Ces résultats sont représentés sur la figure 5.

Au vu de ces résultats, l'intérêt des compositions nutritives selon l'invention dans la production de métabolites et leur supériorité par rapport à celles de l'art antérieur sont clairement démontrés.

## Revendications

1. Composition nutritive résultant de la trempe du maïs, caractérisée par le fait qu'elle présente un rapport des concentrations en phosphore inorganique et phosphore total (Pi/Pt) compris entre 35 et 95 %, et que le dosage des protéines qu'elle contient, réalisé selon un test C, donne une valeur inférieure ou égale à 5.

2. Composition nutritive selon la revendication 1, caractérisée par le fait que son rapport Pi/Pt est compris entre 60 et 95 %, et de préférence entre 75 et 85 %.

3. Composition nutritive selon l'une ou l'autre des revendications 1 et 2, caractérisée par le fait qu'elle présente une matière sèche supérieure ou égale à 60 %.

4. Procédé d'obtention d'une composition nutritive selon la revendication 1, caractérisé par le fait que l'on soumet les eaux de trempage du maïs à un traitement enzymatique à l'aide d'au moins une protéase et d'au moins une phytase.

5. Procédé selon la revendication 3, caractérisé par le fait que le traitement enzymatique consiste :
- à introduire successivement ou simultanément dans l'eau de trempe du maïs au moins une protéase et au moins une phytase,
- à laisser agir les enzymes sous agitation,
- à suivre, par prélèvements, l'évolution au cours du temps du rapport Pi/Pt et de la concentration en protéines,
- à inactiver les enzymes,
- à concentrer la composition résultante par évaporation.

6. Procédé selon l'une ou l'autre des revendications 4 et 5, caractérisé par le fait que le traitement enzymatique s'effectue sur une eau de trempe dont la matière sèche est comprise entre 5 et 50 %, le pH entre 3 et 5, à une température variant entre 40 et 60°C et pendant 4 à 16 heures.

7. Procédé selon l'une quelconque des revendications 4 à 6, caractérisé par le fait que la composition est centrifugée puis concentrée jusqu'à une matière sèche supérieure ou égale à 60 %.

8. Utilisation d'une composition nutritive selon l'une des revendications 1 à 3 comme milieu de culture de microorganismes.

9. Utilisation d'une composition nutritive selon l'une des revendications 1 à 3 en tant qu'aliment ou additif alimentaire dans des compositions destinées aux humains.

10. Utilisation d'une composition nutritive selon l'une des revendications 1 à 3 en tant qu'aliment ou additif alimentaire dans des compositions destinées aux animaux.

## Patentansprüche

1. Nährzusammensetzung, resultierend aus dem Wasser des Einweichens von Mais, dadurch gekennzeichnet, daß sie ein Verhältnis der Konzentrationen von anorganischem Phosphor und Gesamtphosphor (Pi/Pt) zwischen 35 % und 95 % aufweist und daß die Bestimmung der sie enthaltenden Proteine, realisiert nach einem Test C, einen Wert von unterhalb oder gleich 5 ergibt.

2. Nährzusammensetzung nach Anspruch 1, dadurch gekennzeichnet, daß ihr Verhältnis Pi/Pt zwischen 60 % und 95 %, vorzugsweise zwischen 75 % und 85 % liegt.

3. Nährzusammensetzung nach dem einen oder anderen der Ansprüche 1 und 2, dadurch gekennzeichnet, daß sie eine Trockenmasse von höher oder gleich 60 % aufweist.

4. Verfahren zur Herstellung einer Nährzusammensetzung nach Anspruch 1, dadurch gekennzeichnet, daß man die Wässer aus dem Einweichen von Mais einer enzymatischen Behandlung mit Hilfe von mindestens einer Protease und mindestens einer Phytase unterzieht.

5. Verfahren nach Anspruch 4, dadurch gekennzeichnet, daß die enzymatische Behandlung besteht aus:
- dem schrittweisen oder gleichzeitigen Eintragen von mindestens einer Protease und mindestens eine Phytase in das Wasser aus dem Einweichen von Mais,
- dem Einwirkenlassen der Enzyme unter Rühren,
- dem Verfolgen mit Hilfe von Entnanmen im Laufe der Zeit von: der Entwicklung des Verhältnisses (Pi/Pt) und der Konzentration an Proteinen,
- dem Inaktivieren der Enzyme,
- dem Konzentrieren der erhaltenen Zusammensetzung durch Eindampfen.

6. Verfahren nach dem einen oder anderen der Ansprüche 4 und 5, dadurch gekennzeichnet, daß die enzymatische Behandlung mit einem Einweichwasser, dessen Trockenmasse zwischen 5 % und 50 % und dessen pH-Wert zwischen 3 und 5 betragen, sowie bei einer Temperatur zwischen 40 °C und 60 °C und während 4 bis 16 Stunden durchgeführt wird.

7. Verfahren nach irgendeinem der Ansprüche 4 bis 6, dadurch gekennzeichnet, daß die Zusammensetzung zentrifugiert und anschließend bis auf eine Trockenmasse von höher oder gleich 60 % konzentriert wird.

8. Verwendung einer Nährzusammensetzung nach einem der Ansprüche 1 bis 3 als Kulturmedium für Mikroorganismen.

9. Verwendung einer Nährzusammensetzung nach irgendeinem der Ansprüche 1 bis 3 als Nahrungsmittel oder Nahrungszusatzstoff in für den Menschen vorgesehenen Zusammensetzungen.

10. Verwendung einer Nährzusammensetzung nach irgendeinem der Ansprüche 1 bis 3 als Nahrungsmittel oder Nahrungszusatzstoff in für Tiere vorgesehenen Zusammensetzungen.

## Claims

1. Nutrient composition resulting from maize steeping, characterized in that it has an inorganic phosphorus concentration to total phosphorus concentration ratio (Pi/Pt) of between 35 and 95%, and in that the assay of the proteins which it contains, carried out according to a C test, gives a value of less than or equal to 5.

2. Nutrient composition according to Claim 1, characterized in that its Pi/Pt ratio is between 60 and 95%, and preferably between 75 and 85%.

3. Nutrient composition according to either of Claims 1 and 2, characterized in that it has a dry matter content greater than or equal to 60%.

4. Process for producing a nutrient composition according to Claim 1, characterized in that maize steepwater is subjected to an enzymatic treatment with the aid of at least one protease and at least one phytase.

5. Process according to Claim 3, characterized in that the enzymatic treatment consists in:
- introducing successively or simultaneously into the maize steepwater at least one protease and at least one phytase,
- allowing the enzymes to act, with stirring,
- monitoring, by sampling, the variation over time of the Pi/Pt ratio and the protein concentration,
- inactivating the enzymes,
- concentrating the resulting composition by evaporation.

6. Process according to either of Claims 4 and 5, characterized in that the enzymatic treatment is performed on a steepwater whose dry matter content is between 5 and 50%, and pH between 3 and 5, at a temperature varying between 40 and 60°C and for 4 to 16 hours.

7. Process according to any one of Claims 4 to 6, characterized in that the composition is centrifuged and then concentrated to a dry matter content greater than or equal to 60%.

8. Use of a nutrient composition according to one of Claims 1 to 3, as medium for culturing microorganisms.

9. Use of a nutrient composition according to one of Claims 1 to 3, as food or food additive in compositions intended for humans.

10. Use of a nutrient composition according to one of Claims 1 to 3, as food or food additive in compositions intended for animals.
